(19) Europäisches Patentamt
European Patent Office
Office européen des brevets

(11) EP 2 637 695 B1

(12) EUROPEAN PATENT SPECIFICATION

(45) Date of publication and mention of the grant of the patent:
**13.12.2017 Bulletin 2017/50**

(21) Application number: **11754742.2**

(22) Date of filing: **08.08.2011**

(51) Int Cl.:
***A61K 47/10*** (2017.01) ***A61K 9/20*** (2006.01)
***A61K 9/16*** (2006.01) ***A61K 9/14*** (2006.01)
***A61K 9/50*** (2006.01) ***A61K 31/4178*** (2006.01)
***A61K 45/06*** (2006.01)

(86) International application number:
**PCT/IB2011/053532**

(87) International publication number:
**WO 2012/020368 (16.02.2012 Gazette 2012/07)**

(54) **OLMESARTAN FORMULATIONS**

OLMERSATANFORMULIERUNGEN

FORMULATIONS D'OLMESARTAN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2010 EP 10172244**

(43) Date of publication of application:
**18.09.2013 Bulletin 2013/38**

(73) Proprietor: **Abdi Ibrahim Ilac Sanayi ve Ticaret Anonim Sirketi**
**34555 Istanbul (TR)**

(72) Inventors:
- **FARSHI, Farhad**
  **34555 Istanbul (TR)**
- **SOYLEMEZ, Serdar**
  **34555 Istanbul (TR)**
- **AVCI, Recep**
  **34555 Istanbul (TR)**
- **YILDIRIM, Ersin**
  **34555 Istanbul (TR)**

(56) References cited:
**EP-A1- 2 033 643** **WO-A1-2008/032107**
**WO-A1-2009/084040** **WO-A2-2007/001065**
**WO-A2-2009/110010** **US-A1- 2010 119 607**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 637 695 B1

## Description

### Technical Field

The present invention provides pharmaceutical compositions comprising olmesartan medoxomil in combination with amlodipine besylate and suitable additives for an improved dissolution profiles.

### Background Art

Angiotensin II is a very potent chemical that causes muscles surrounding blood vessels to contract, thereby narrowing blood vessels. This narrowing increases the pressure within the vessels and can cause hypertension. Angiotensin II receptor blockers (ARBs) are medications that block the action of angiotensin II by preventing angiotensin II from binding to angiotensin II receptors on blood vessels. As a result, blood vessels enlarge (dilate) and blood pressure is reduced. Reduced blood pressure makes it easier for the heart to pump blood and can improve heart failure.

Drugs in this class include candesartan, eprosartan, losartan, olmesartan, telmisartan ,valsartan and pratosartan. ARBs are used alone or in combination with other classes of antihypertensive agents that include thiazide diuretics, blockers, calcium channel blockers, rennin inhibitor, and ACE inhibitors, both for the treatment of hypertension and congestive heart failure.

Olmesartan which is an angiotensin II receptor antagonist is pharmaceutically used as an antihypertensive for the treatment and prophylaxis of hypertension.

Its chemical name is 2,3-dihydroxy-2-butenyl 4-(1-hydroxy-1-methylethyl)-2-propyl-1-[j!7-(o-1H-tetrazol-5-ylphenyl)benzyl]imidazol e-5-carboxylate, cyclic 2,3- carbonate or (5-methyl-2-oxo-1,3-dioxolen-4-yl)rnethyl 4-(1-hydroxy-1-methylethyl)-2-propyl- 1-{4-[2-(tetrazol-5-yl)phenyl]phenyl}methylimidazole-5-carboxylate having the following structure:

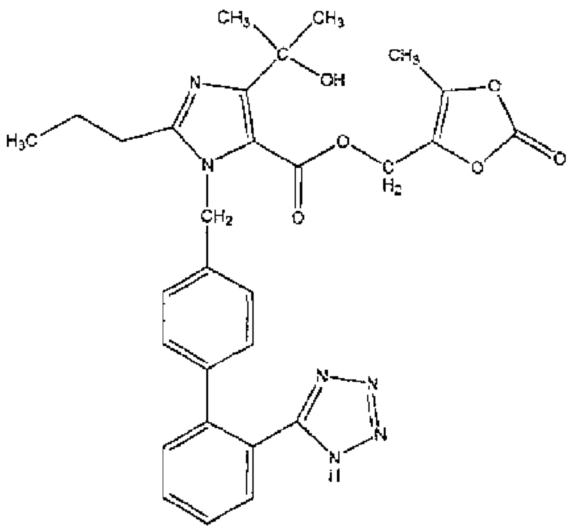

Olmesartan medoxomil was developed to achieve better oral bioavailability.

Olmesartan medoxomil is an ester prodrug of olmesartan which after ingestion liberates the only active metabolite. Olmesartan medoxomil is marketed by Sankyo under the trade name of Olmetec® or Benicar®. It is available as oral tablets in strengths of 5 mg, 10 mg, 20 mg and 40 mg.

Olmesartan medoxomil is practically insoluble in water and in the pH range of 3 to 9, sparingly soluble in strong acid (except insoluble in 17 hydrochloric acid), and soluble in strong base.

Amlodipine is a calcium channel blocker developed for the treatment of hypertension and other medical indications. Its chemical name is

3-ethyl-5-methyl-(±)-2-[(2-aminoethoxy)methyl]-4-(2-chlorophenyl)-1,4-dihydro-6-me thylpyridine-3,5-dicarboxylate, having the following structure:

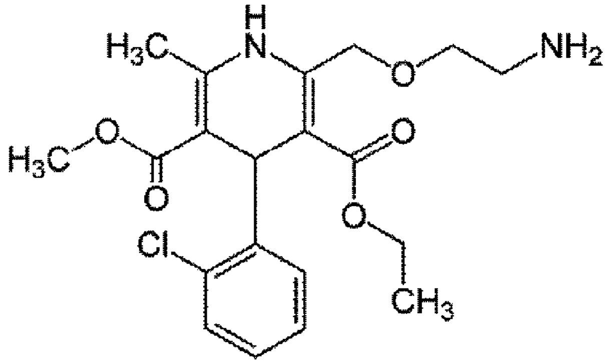

Certain solid pharmaceutical composition comprising olmesartan or pharmaceutically acceptable salts and esters thereof and process for their preparation are known.

Olmesartan was firstly disclosed by EP0503785B (SANKYO COMPANY LIMITED) 16.09.1992.

Amlodipine was firstly disclosed by EP244944 B (PFIZER LIMITED) 11.11.1987.

Olmesartan was firstly disclosed by EP0503785 B (SANKYO COMPANY LIMITED) 16.09.1992.

Amlodipine was firstly disclosed by EP244944 B (PFIZER LIMITED) 11.11.1987.

Olmesartan medoxomil has a low bioavailability (BA), approximately 26% in humans, due to its low water solubility and efflux by drug resistance pumps in the gastrointestinal tract.

The production of drug products containing olmesartan requires formulation techniques which enhance the dissolution property of olmesartan.

Published WO2008/149338A (DEXCEL LTD.) 11.12.2008 discloses a novel method of preparation of a formulation comprising an angiotensin II receptor antagonist and in particular, to a method using melt granulation of solid components. In the pharmaceutical industry, the melt granulation technique has been used for improving the dissolution rate and bioavailability of a drug by forming a solid dispersion or solid solution.

Published EP2033643A (DAIICHI SANKYO LIMITED) 03.01.2008 discloses a production method olmesartan medoxomil containing drug product having an improved dissolution property.

Published WO 2007/001066A (SANKYO COMPANY LIMITED) 04.01.2007 discloses a pharmaceutical preparation comprising an angiotensin II receptor antagonist and a calcium channel blocker. A pharmaceutical preparation with improved dissolution properties is obtained by incorporation of at least one substance selected from a hydrophilic polymer, an acidic substance and a fluiziding agent.

Published WO 2007/001065 (SANKYO COMPANY LIMITED) 04.01.2007 discloses a solid dosage pharmaceutical preparation comprising an angiotensin II receptor antagonist, a calcium channel blocker and at least one hydrophilic polymer with improved dissolution properties, the said dosage form is prepared by wet granulation.

Published WO2008/032107 (SANKYO COMPANY LIMITED) 20.03.2008 discloses a solid dosage pharmaceutical preparation comprising olmesartan medoxomil and amlodipine or pharmaceutically acceptable salt thereof with improved stability of active ingredients, said dosage form is substantially free reducing sugars.

Published US 2010/0119607 (Rubicon Research Pvl. Ltd.) 13.05.2010 discloses amethod of increasing the bioavailability of an angiotensin II receptor blockers by preparing a composition of angiotensin I receptor blockers with at least one solubility enhancing agent.

Published WO 2009/084040 (Rubicon Research Pvt. Ltd.) 09.07.2009 discloses once day formulation comprising solubilizing an angiotensin II receptor blocker with solubility enhancing agent.

## Summary of invention

It is an object of the present invention to provide a pharmaceutical composition comprising: olmesartan medoxomil in the range of from 5% to 45%, amlodipine besylate in the range of from 2% to 20%, poloxamer in the range of from 0.1 % to 15%, disintegrant in the range of from 1% to 15%, filler/diluent in the range of from 5% to 80%, glidant in the range of from 0.1 % to 10%, lubricant in the range of from 0.1 % to 5%, coating agent in the range of from 0.1 % to 10% by weight of total pharmaceutical composition prepared by direct compression or wet granulation methods. It is also an object of the present disclosure to provide a process according to claim 10 or claim 11 for preparing a solid pharmaceutical composition comprising olmesartan medoxomil and amlodipine besylate in combination wherein the solubility and bioavailability of olmesartan is improved with a mechanical process of mixing and sieving of active ingredient with poloxamer by using a screen of 315 microns.

In the present invention poloxamer is used as a solubility enhancer agent in a spesific amount of 0.1-15 %(w/w) of the total pharmaceutical composition.

Technical Problem

In drug development, therapeutic effectiveness of a drug depends upon the bioavailability and ultimately upon the solubility of drug molecules. Bioavailability is a pharmacokinetic parameter defined by the amount of a drug absorbed based on the amount administered, which is used to determine the effectiveness of an administered pharmaceutical active ingredient or a formulation comprising same. The characteristics of a pharmaceutical active ingredient, e.g., water-solubility, crystal forms and particle size of the active ingredient can affect the bioavailability of the active ingredient or a composition comprising same. Low water solubility and low bioavailability are frequent problems which arc directly related to the drug molecule and need to be solved. For example, the sparingly water-soluble drug itself is not absorbed in the gastrointestinal tract, leading to poor bioavailability.
Various techniques are available to improve the solubility of poorly soluble drugs. Chemical and physical properties of active ingredient can be modified.
Besides active ingredient's physical and chemical modifications, different formulation techniques which enhance the dissolution property of the active ingredient are generally used.
Although use of solvent recrystallization, inclusion in cyclodextrin- drug complexes and novel technologies such as super critical fluid processing, micronization via nanoparticles are widespread possess significant limitations by means of productions.
Published PCT application WO 2008/013416 A (AMOREPACIFIC CORPORATION) 31.01.2008 discloses a process for preparing a powder composition comprising nanoparticles of a sparingly water-soluble drug, which exhibits enhanced bioavailability and particle size stability of the drug, when dispersed in an aqueous medium.

**Solution to Problem**

In order to solve low bioavailability and low solubility of drug molecule, it is invented that olmesartan medoxomil, poloxamer and preferably futher excipient or mixtures of excipients are intimately admixtured. Olmesartan medoxomil is mechanically treated and sieved with poloxamer in terms of admixturing. Poloxamer which is used in the range of 0.1%- 15 %(w/w) of total pharmaceutical composition improve drug-membran interaction. This method does not require any supplemental investment or expenses.

**Description of embodiments**

In the present invention, inventors provide a pharmaceutical formulation and a production method to get a better bioavailability, solubility property by a reproducible, simple manufacturing method.
In the first aspect of this invention, olmesartan medoxomil is treated with poloxamer. Treatment can be numerous manners whatsoever. The preparation method of pharmaceutical composition of olmesartan medoxomil in combination with amlodipine besylate is characterized in that: a-) an intimate admixture of olmesartan medoxomil and the solubility enhancing agent poloxamer is prepared and b-) said solubility enhancing agent is used in the range of 0.1%-15% by total weight of pharmaceutical composition. An intimate admixture can be obtained, for example, by co-sieved, co-precipitation, co-milling, compression, granulation, or the like.
Poloxamers are water-soluble, nonionic, triblock copolymeric surfactants of poly(ethylene oxide) and poly(propylene oxide) .Among them, poloxamer 188 was approved by the Food and Drug Administration as a safe ingredient, it is reported in the National Formulary as a pharmaceutical ingredient. USP/NF specifies 5 different types of poloxamers with different chain lengths for *a* and *b*.

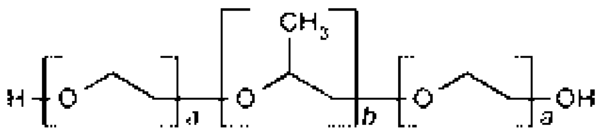

In solid preparations, poloxamer acts as wetting agent, plasticizer and solubility enhancing agent to improve the solubility and bioavailability of sparingly water soluble active drugs.
In this invention, olmesartan medoxomil and poloxamer particles are sized with a screen such as 315 microns but less than 710 microns. The drug is in intimate contact with the poloxamer in the particles. By this method, bioavailability of olmesartan is improved by increasing the surface area which enhances the bioavailability of olmesartan.
Olmesartan and poloxamer are sieved from a suitable milling equipment of preferably 315 microns. As an example, fitzmill enables comminution to a spesific particle range of active ingredient. By this process, active ingredient particles surface area is increased which helps dissolution and permeability of active ingredient. Furthermore, active

ingredient particles are surrounded with poloxamer which improve the dissolution properties of drug as well.

In solid preparations, poloxamer acts as wetting agent, plasticizer and solubility enhancing agent to improve the solubility and bioavailability of sparingly water soluble active drugs.

According to this invention, poloxamer is used in an amount of 0.1%-15% (w /w) of total tablet weight is found to be optimum amount to improve the solubility, absorption and bioavailability of active ingredient.

In order to obtain better dissolution results, active ingredient and poloxamer mixtures are sized with different screens. Production by direct compression clearly showed that sifting low bio available active ingredient with poloxamer from a preferably 315 microns sieve enhanced the solubility of drug.

Sized from preferably 315 microns screen, particles show better dissolution results compared to particles sized with 710 microns screen.

The dissolution profiles results obtained from different screens are summarized below.

[Table 1]

| Time (Minutes) | Dissolved % | | |
|---|---|---|---|
| | **Sevikar® Daiichi Sankyo (Reference)** | **Olmesartan Containing Film Tablet wherein olmesartan and poloxamer sieved with 315 microns screen (Test)** | **Olmesartan Containing Film Tablet wherein olmesartan and poloxamer sieved with 710 microns screen (Test)** |
| 10 | 58,9 | 56,8 | 49,6 |
| 15 | 65,6 | 65,9 | 59,6 |
| 20 | 70,9 | 72,3 | 66,0 |
| 30 | 76,8 | 78,5 | 73,1 |
| 45 | 81,8 | 82,6 | 78,7 |

Similarity factor $F_2$ value is found 89.1 for olmesartan containing film tablet wherein olmesartan medoxomil and poloxamer are sized by 315 microns screen when compared to the reference product. $F_2$ value is 60.5 for olmesartan containing film tablet wherein olmesartan medoxomil and poloxamer are sized by 710 microns screen when compared to the reference product. It shows that increased surface area of drug molecule which is treated with poloxamer improves the dissolution properties and bioavailability of the drug.

Thus an another aspect of this invention is that olmesartan medoxomil and poloxamer particles are sized with a screen less than 710 microns preferably 315 microns in terms of intimately admixturing.

According to this invention, the pharmaceutical compositions of olmesartan and active pharmaceutical ingredient or ingredients wherein formulations can be prepared by wet granulation, dry granulation, direct compression and the like. Said pharmaceutical composition may further comprise pharmaceutically acceptable excipients or excipients selected from the group consisting of diluents, binders, disintegrants, fillers, solubility enhancing agents, lubricants, glidants, mixtures thereof and the like.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches (including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are,

but not limited to, colloidal silica, silicon dioxide, powdered cellulose, talc, tribasic calcium phosphate, mixtures thereof and the like.

Preferably, the solubility enhancing agent may be PEG-20-glyceryl stearate (Capmul® by Abitec), PEG-40 hydrogenated castor oil (Cremophor RH 40® by BASF), PEG 6 corn oil (Labrafil® by Gattefosse), lauryl macrogol - 32 glyceride (Gelucire 44/14® by Gattefosse), stearoyl macrogol glyceride (Gelucire 50/13® by Gattefosse), polyglyceryl - 10 mono dioleate (Caprol ® PEG 860 by Abitec), propylene glycol oleate (Lutrol OP® by BASF), propylene glycol dioctanoate (Captex® by Abitec), propylene glycol caprylate/caprate (Labrafac® by Gattefosse), glyceryl monooleate (Peceol® by Gattefosse), glycerol monolinoleate (Maisine ® by Gattefosse), glycerol monostearate (Capmul® by Abitec), PEG- 20 sorbitan monolaurate (Tween 20® by ICI), PEG - 4 lauryl ether (Brij 30® by ICI), sucrose distearate (Sucroester 7® by Gattefosse), sucrose monopalmitate (Sucroester 15® by Gattefosse) ,polyoxyethylene-polyoxypropylene block copolymer (Lutrol® series BASF), polyethylene glycol 660 hydroxystearate, (Solutol® by BASF), sodium lauryl sulphate, sodium dodecyl sulphate, dioctyl suphosuccinate, L- hydroxypropyl cellulose, hydroxylethylcellulose, hydroxy propylcellulose, propylene glycol alginate, sodium taurocholate, sodium glycocholate, sodium deoxycholate, betains , polyethylene glycol (Carbowax® by DOW), d-α- tocopheryl polyethylene glycol 1000 succinate (Vitamin E TPGS® by Eastman), or mixtures thereof.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

According to this invention, a pharmaceutical composition comprising olmesartan or pharmaceutically acceptable salt and esters, one or more active ingredient and poloxamer can also be prepared by direct compression.

In an embodiment of this invention, prepared by direct compression method, direct compression encompasses olmesartan or pharmaceutically acceptable salts and esters thereof and amlodipine or pharmaceutically acceptable salts thereof. Accordingly, combination comprises; olmesartan medoxomil is in the range of from about 5% to about 45%, amlodipine besylate is in the range of from about 2% to about 20%, poloxamer is in the range of from about 0.1% to about 15%, disintegrant is in the range of from about 1% to about 15%, filler/diluent is in the range of from about 5% to about 80%, glidant is in the range of from about 0.1% to about 10%, lubricant is in the range of from about 0.1% to about 5%, coating agent is in the range of from about 0.1% to about 10% by weight of total pharmaceutical composition.

Preferably, pharmaceutical composition comprising olmesartan medoxomil is 19.42%, amlodipine besylate is 6.73% , poloxamer is 2.00%, disintegrant is 4.85%, filler/diluent is 61.18 %, glidant is 2.18%, lubricant is 0.73%, coating agent is 2.92% % by the weight of total pharmaceutical composition.

[Table 2]

| **Total Pharmaceutical Composition (40/10)** | | |
|---|---|---|
| **Ingredients** | **mg** | **%** |
| Olmesartan Medoxomil | 40,00 | 19,42 |
| Amlodipine Besylate | 13,86 | 6,73 |
| Poloxamer | 4,12 | 2,00 |
| Filler/Diluent | 126,02 | 61,18 |
| Disintegrant | 10,00 | 4,85 |
| Glidant | 4,50 | 2,18 |
| Lubricant | 1,50 | 0,73 |
| Coating Agent | 6,00 | 2,92 |
| **Total** | **206,00** | **100,00** |

On the other hand this invention includes a preparation method for direct compression of olmesartan or pharmaceutically acceptable salts and esters and amlodipine or pharmaceutically acceptable salts. This method comprising steps of a. Olmesartan medoxomil and poloxamer are sieved by using a screen of 315 microns and installed into container and mixed. b. A portion of filler/diluent is added to powder prepared at step (a) and mixed. c. Amlodipine besylate, a portion of filler are sieved by using suitable equipment and mixed. d. A portion of filler/diluent and disintegrant are added

to the mixture prepared at step (b) e. The mixture prepared at step (c) and (d) are mixed f. Pre-sieved lubricant and pre-sieved glidant are installed into container onto powder mixture prepared at step e and mixed. g. The final mixture is compressed h. A Coating agent or mixtures of coating agents are added into solvent and stirred. i. Core tablets are installed into a coating pan and coated with the coating suspension prepared at step h.

According to the present invention, pharmaceutical compositions are preferably prepared by a wet granulation process.

In an embodiment of this invention, the process for preparing solid oral dosage forms comprising combination of olmesartan or pharmaceutically acceptable salts and esters thereof and amlodipine or pharmaceutically acceptable salts wherein composition has intra-granulation step and intra-granular ingredient(s) and extra-granulation step and extra-granular ingredient(s) .It comprises steps of:

**a. Intragranulation**

1. Olmesartan medoxomil and poloxamer are mixed and sieved by using a screen of 315 microns.
2. Binder and solvent are mixed until binder is dissolved.
3. Mixture prepared at step (1), amlodipine besylate, filler and a portion of disintegrant are mixed in the granulator.
4. The granulation solution prepared at step 2 is added onto the mixture in the granulator to form granules.
5. The blend is mixed and transferred to a fluidized bed dryer until proper moisture then sizing the dried granules by using suitable milling equipment.

**b.Extragranulation**

1. Granules of internal phase are transferred to the mixer, a portion of disintegrant and additional filler are added to the mixture.
2. Pre-sieved glidant and lubricant are added to the mixture and mixed.
3. The final blend is compressed on a tabletting machine using appropriate punches.
4. Coating agent or mixtures of coating agents are added into purified water and stirred.
5. Core tablets are installed into a coating pan and coated with the coating suspension prepared at step (4).

In a further embodiment, solvents used for granulation process may be selected from water, isopropyl alcohol, acetone, ethanol, methylene chloride mixtures thereof and the like.

In another aspect of this invention, intragranulation comprising olmesartan medoxomil is from about 5% to about 45%, amlodipine besylate is from about 2% to about 20%, poloxamer is from about 0.1 % to about 15%, filler/diluent is from about 5% to about 80%, disintegrant is from about 1% to about 15%, and binder is from about 0.1% to about 5% by weight of intragranular phase's ingredients.

Preferably, intragranulation comprising olmesartan medoxomil is 31.24%, amlodipine besylate is 10.82%, poloxamer is 3.28%, filler/diluent is 46.85%, disintegrant is 4.69%, binder is 3.12% by total weight of intragranulation phase's ingredients.

[Table 3]

| **Intragranulation (40/10-20/5)** | | |
|---|---|---|
| **Ingredients** | **mg** | **%** |
| Olmesartan Medoxomil | 40,00 | 31,24 |
| Amlodipine Besylate | 13,86 | 10,82 |
| Poloxamer | 4,20 | 3,28 |
| Binder | 4,00 | 3,12 |
| Filler | 60,00 | 46,85 |
| Disintegrant | 6,00 | 4,69 |
| **Total** | 128,06 | 100,00 |

Extragranulation comprising disintegrant is from about 1% to about 15%, filler/ diluent is from about 5% to about 80%, glidant is from about 0.1% to about 10%, lubricant is from about 0.1% to about 5%, and coating agent is from about 0.1% to about 10 by total weight of extragranular phase's excipients.

Preferably, extragranulation comprising disintegrant is 7.70%, filler is 76.98%, glidant is 5.70%, lubricant is 1.92 %, coating agent is 7.70% by total weight of extra-granulation step's excipients.

[Table 4]

| **Extragranulation(40/10-20/5)** | | |
|---|---|---|
| **Ingredients** | **mg** | **%** |
| Disintegrant | 6,00 | 7,70 |
| Filler/Diluent | 60,00 | 76,98 |
| Glidant | 4,44 | 5,70 |
| Lubricant | 1,50 | 1,92 |
| Coating Agent | 6,00 | 7,70 |
| **Total** | 77,94 | 100,00 |

In an embodiment of this invention, olmesartan medoxomil is in the range of from about %5 to about 45%, amlodipine besylate is in the range of from about %2 to about 20%, poloxamer is in the range of from about 0.1% to about 15%, binder is in the range of from about %0.1 to about 5%, disintegrant is in the range of from about 1% to about 15%, filler/diluent is in the range of from about 5% to about 80%, glidant is in the range of from about 0.1% to about 10%, lubricant is in the range of from about 0.1% to about 5%, coating agent is in the range of from about 0.1% to about 10% by weight of total pharmaceutical composition.

Preferably, pharmaceutical composition comprising olmesartan medoxomil thereof is 19.42%, amlodipine besylate is 6.73%, poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition.

[Table 5]

| **Total Pharmaceutical Composition (40/10-20/5)** | | |
|---|---|---|
| **Ingredients** | **mg** | **%** |
| Olmesartan Medoxomil | 40,00 | 19,42 |
| Amlodipine Besylate | 13,86 | 6,73 |
| Poloxamer | 4,20 | 2,04 |
| Binder | 4,00 | 1,94 |
| Disintegrant | 12,00 | 5,82 |
| Filler/Diluent | 120,00 | 58,26 |
| Glidant | 4,44 | 2,16 |
| Lubricant | 1,50 | 0,73 |
| Coating Agent | 6,00 | 2,91 |
| **Total** | **206,00** | **100,00** |

In another aspect of this invention, in 40 mg Olmesartan Medoxomil /5 mg amlodipine besylate formulations, intragranulation comprising olmesartan medoxomil is from about 5% to about 45%, amlodipine besylate is from about 2% to about 20%, poloxamer is from about 0.1% to about 15%, filler/diluent is from about 5% to about 80%, disintegrant is from about 1% to about 15% and binder is from about 0.1% to about 5% by weight of intragranular phase's ingredients.

Preferably, in 40 mg olmesartan medoxomil /5 mg amlodipine besylate formulations, intragranulation comprising olmesartan medoxomil is 33.02%, amlodipine besylate is 5.72%, poloxamer is 3.47%, filler/diluent is 49.53%, disintegrant is 4.95%, binder is 3.30% by total weight of intragranulation phase's ingredients.

[Table 6]

| Intragranulation (40/5) | | |
|---|---|---|
| **Ingredients** | **mg** | **%** |
| Olmesartan Medoxomil | 40,00 | 33,02 |
| Amlodipine Besylate | 6,93 | 5,72 |
| Poloxamer | 4,20 | 3,47 |
| Binder | 4,00 | 3,30 |
| Filler/Diluent | 60,00 | 49,53 |
| Disintegrant | 6,00 | 4,95 |
| **Total** | **121,13** | **100,00** |

In 40 mg Olmesartan medoxomil /5 mg amlodipine besylate formulations, extra-granulation comprising disintegrant is from about 1% to about 15%, filler/diluent is from about 5% to about 80%, glidant is from about 0.1% to about 10 %, lubricant is from about 0.1% to about 5%, and coating agent is from about 0.1% to about 10 by total weight of extragranular phase's excipients.

In 40 mg Olmesartan Medoxomil /5 mg amlodipine besylate formulations, extra-granulation preferably comprising disintegrant is 7.07%, filler is 78.86%, glidant is 5.23%, lubricant is 1.77 %, coating agent is 7.07% by total weight of extragranulation step's excipients.

[Table 7]

| Extragranulation(40/5) | | |
|---|---|---|
| **Ingredients** | **mg** | **%** |
| Disintegrant | 6,00 | 7,07 |
| Filler/Diluent | 66,93 | 78,86 |
| Glidant | 4,44 | 5,23 |
| Lubricant | 1,50 | 1,77 |
| Coating Agent | 6,00 | 7,07 |
| **Total** | **84,87** | **100,00** |

In 40 mg olmesartan medoxomil /5 mg amlodipine besylate formulations, olmesartan medoxomil is in the range of from about %5 to about 45%, amlodipine besylate is in the range of from about %2 to about 20%, poloxamer is in the range of from about 0.1% to about 15%, binder is in the range of from about % 0.1 to about 5%, disintegrant is in the range of from about 1% to about 15%, filler/diluent is in the range of from about 5% to about 80%, glidant is in the range of from about 0.1% to about 10%, lubricant is in the range of from about 0.1% to about 5%, coating agent is in the range of from about 0.1% to about 10% by weight of total pharmaceutical composition.

In 40 mg olmesartan medoxomil /5 mg amlodipine besylate formulations, pharmaceutical composition preferably comprising olmesartan medoxomilthereof is 19.42%, amlodipine besylate is 3.36% ,poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition.

[Table 8]

| Total Pharmaceutical Composition (40/5) | | |
|---|---|---|
| **Ingredients** | **mg** | **%** |
| Olmesartan Medoxomil | 40,00 | 19,42 |
| Amlodipine Besylate | 6,93 | 3,36 |
| Poloxamer | 4,20 | 2,04 |

(continued)

| Total Pharmaceutical Composition (40/5) | | |
|---|---|---|
| **Ingredients** | **mg** | **%** |
| Binder | 4,00 | 1,94 |
| Disintegrant | 12,00 | 5,82 |
| Filler/Diluent | 126,93 | 58,26 |
| Glidant | 4,44 | 2,16 |
| Lubricant | 1,50 | 0,73 |
| Coating Agent | 6,00 | 2,91 |
| **Total** | **206,00** | **100,00** |

In another aspect of this invention, in 20 mg Olmesartan Medoxomil /10 mg amlodipine besylate formulations, intragranulation comprising olmesartan medoxomil is from about 5% to about 45%, amlodipine besylate is from about 2% to about 20%, poloxamer is from about 0.1% to about 15%, filler/diluent is from about 5% to about 80% disintegrant is from about 1% to about 15% and binder is from about 0.1% to about 5% by weight of intragranular phase's ingredients.
In 20 mg Olmesartan Medoxomil /10 mg amlodipine besylate formulations ,preferably, intragranulation comprising olmesartan medoxomil is 16.94%, amlodipine besylate is 11.74%, poloxamer is 1.78%, filler/diluent is 61.07%, disintegrant is 5.08%, binder is 3.39% by total weight of intragranulation phase's ingredients.

[Table 9]

| Intragranulation (20/10) | | |
|---|---|---|
| **Ingredients** | **mg** | **%** |
| Olmesartan Medoxomil | 20,00 | 16,94 |
| Amlodipine Besylate | 13,86 | 11,74 |
| Poloxamer | 2,10 | 1,78 |
| Binder | 4,00 | 3,39 |
| Filler/Diluent | 72,10 | 61,07 |
| Disintegrant | 6,00 | 5,08 |
| **Total** | **118,06** | **100,00** |

In an another aspect of this invention, in 20 mg olmesartan medoxomil /10 mg amlodipine besylate formulations, extragranulation comprising disintegrant is from about 1% to about 15%, filler/diluent is from about 5% to about 80%, glidant is from about 0.1% to about 10%, lubricant is from about 0.1% to about 5%, and coating agent is from about 0.1% to about 10% by total weight of extragranular phase's excipients.
In 20 mg olmesartan medoxomil /10 mg amlodipine besylate formulations, preferably, extragranulation comprising disintegrant is 6.82%, filler/diluent is 79.60%, glidant is 5.05%, lubricant is 1.71 %, coating agent is 6.82% by total weight of extra-granulation step's excipients.

[Table 10]

| Extragranulation(20/10) | | |
|---|---|---|
| **Ingredients** | **mg** | **%** |
| Disintegrant | 6,00 | 6,82 |
| Filler/Diluent | 70,00 | 79,60 |
| Glidant | 4,44 | 5,05 |
| Lubricant | 1,50 | 1,71 |

(continued)

| Extragranulation(20/10) | | |
|---|---|---|
| **Ingredients** | **mg** | **%** |
| Coating Agent | 6,00 | 6,82 |
| **Total** | **87,94** | **100,00** |

In an embodiment of this invention, in aspect of 20 mg olmesartan medoxomil /10 mg amlodipine besylate formulations, olmesartan medoxomil is in the range of from about %5 to about 45%, amlodipine besylate is in the range of from about %2 to about 20%, poloxamer is in the range of from about 0.1% to about 15%, binder is in the range of from about %0.1 to about 5%, disintegrant is in the range of from about 1% to about 15%, filler/diluent is in the range of from about 5% to about 80%, glidant is in the range of from about 0.1% to about 10%, lubricant is in the range of from about 0.1% to about 5%, coating agent is in the range of from about 0.1% to about 10% by weight of total pharmaceutical composition.

In 20 mg Olmesartan Medoxomil /10 mg amlodipine besylate formulations,preferably, pharmaceutical composition comprising olmesartan medoxomil is 9.71%, amlodipine besylate is 6.73% , poloxamer is 1.02%, binder is 1.94%, disintegrant is 5.82%, filler/diluent is 68.98 %, glidant is 2.16%, lubricant is 0.73%, coating agent is 2.91 % by the weight of total pharmaceutical composition.

[Table 11]

| Total Pharmaceutical Composition (20/10) | | |
|---|---|---|
| **Ingredients** | **mg** | **%** |
| Olmesartan Medoxomil | 20,00 | 9,71 |
| Amlodipine Besylate | 13,86 | 6,73 |
| Poloxamer | 2,10 | 1,02 |
| Binder | 4,00 | 1,94 |
| Disintegrant | 12,00 | 5,82 |
| Filler/Diluent | 142,10 | 68,98 |
| Glidant | 4,44 | 2,16 |
| Lubricant | 1,50 | 0,73 |
| Coating Agent | 6,00 | 2,91 |
| **Total** | **206,00** | **100,00** |

A suitable dissolution method is used to reach dissolution profiles. In this invention USP Type-2 apparatus is preferred and used. According to the method which was developed during drug development process, the tablets prepared in accordance with the present invention should exhibit the following dissolution profile when tested in a USP Type 2 apparatus, at 50 rpm, and 37° C,900 ml and in pH=6,8 phosphate buffer medium.

To determine the similarity between the dissolution profiles of the test and reference product a simple model independent approach, that is $f_2$ (similarity factor), was carried out. As per US FDA, $f_2$ values should lie between 50 and 100 for developing two similar dissolution profiles.

The similarity factor *f2* is a measurement of the similarity through a point by point comparison as shown in Equation 1.

Equation

$$f_2 = 50 * \log \frac{100}{\sqrt{1 + \frac{1}{n}\sum_{t=1}^{n}(R_t - T_t)^2}}$$

n: is the number of sampling time points
Rt : is the amount drug released from a reference batch at time t
Tt : is the amount drug released from a test batch at time t.

Generally, f2 values greater than 50 ensure sameness of the performance of the reference product and test product.

The test product and the reference product's results show that the $f_2$ is in the limits of 50- 100 as established by the US FDA for claiming similarity between the dissolution profiles of the test and reference product.

Olmesartan medoxomil test tablet is released in pH=6,8 phosphate buffer environment under conditions of 900 ml of a dissolution medium at 37 C, USP method-II (pedal) 50 rpm wherein tablet exhibits a dissolution profile.(Table 12, Figure 1).$F_2$ value is 98.7.

The results obtained by wet granulation method are summarized below table.

[Table 12]

| Time (Minutes) | Dissolved % | |
|---|---|---|
| | Sevikar® Daiichi Sankyo (Reference) | Olmesartan Film Tablet (Test) |
| 10 | 60,6 | 60,6 |
| 15 | 68,1 | 68,5 |
| 20 | 72,8 | 72,7 |
| 30 | 78,8 | 78,2 |
| 45 | 82,3 | 82,1 |

## Claims

1. A pharmaceutical composition comprising: olmesartan medoxomil is in the range of from 5% to 45%, amlodipine besylate is in the range of from 2% to 20%, poloxamer is in the range of from 0.1 % to 15%, disintegrant is in the range of from 1% to 15%, filler/diluent is in the range of from 5% to 80%, glidant is in the range of from 0.1 % to 10%, lubricant is in the range of from 0.1 % to 5%, coating agent is in the range of from 0.1 % to 10% by weight of total pharmaceutical composition prepared by direct compression or wet granulation methods.

2. A pharmaceutical composition according to claim 1 comprising: olmesartan medoxomil is 19.42%, amlodipine besylate is 6.73%, poloxamer is 2.00%, disintegrant is 4.85%, filler/diluent is 61.18 %, glidant is 2.18%, lubricant is 0.73%, coating agent is 2.92% by the weight of total pharmaceutical composition.

3. A pharmaceutical composition according to claim 1, prepared by wet granulation method wherein pharmaceutical composition comprising : olmesartan medoxomil is in the range of from 5% to 45%, amlodipine besylate is in the range of from 2% to 20%, poloxamer is in the range of from 0.1 % to 15%, binder is in the range of from 0.1% to 5%, disintegrant is in the range of from 1% to 15%, filler/diluent is in the range of from 5% to 80%, glidant is in the range of from 0.1 % to 10%, lubricant is in the range of from 0.1 % to 5%, coating agent is in the range of from 0.1 % to 10% by weight of total pharmaceutical composition.

4. A pharmaceutical composition according to claim 3, a-) in 40 mg Olmesartan Medoxomil/10 mg amlodipine besylate and in 20 mg Olmesartan Medoxomil /5 mg amlodipine besylate formulations, pharmaceutical composition comprising:

   olmesartan medoxomil is 19.42%, amlodipine besylate is 6.73%, poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition, b-) In 40 mg Olmesartan Medoxomil/5mg amlodipine besylate formulations, pharmaceutical composition comprising : olmesartan medoxomil is 19.42%, amlodipine besylate is 3.36% ,poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition, c-). In 20 mg Olmesartan Medoxomil/10 mg amlodipine besylate formulations, pharmaceutical composition comprising:

olmesartan medoxomil is 9.71 %, amlodipine besylate is 6.73% , poloxamer is 1.02%, binder is 1.94%, disintegrant is 5.82%, filler/diluent is 68.98 %, glidant is 2.16%, lubricant is 0.73%, coating agent is 2.91 % by the weight of total pharmaceutical composition.

**5.** A pharmaceutical composition according to claim 3, wherein said wet granulation method comprises intragranulation and extragranulation steps.

**6.** A pharmaceutical composition according to claim 5, wherein said intragranulation step comprises: olmesartan medoxomil is in the range of from 5% to 45%, amlodipine besylate is in the range of from 2% to 20%, poloxamer is in the range of from 0.1 % to 15%, filler/diluent is in the range of from 5% to 80% disintegrant is in the range of from 1% to 15% and binder is in the range of from 0.1 % to 5% by weight of intragranular phase's ingredients.

**7.** A pharmaceutical composition according to claim 6, a-) in 40 mg Olmesartan Medoxomil/10 mg amlodipine besylate and in 20 mg Olmesartan Medoxomil /5 mg amlodipine besylate formulations, intragranulation comprising: olmesartan medoxomil is 31.24%, amlodipine besylate is 10.82%, poloxamer is 3.28%, filler/diluent is 46.85%, disintegrant is 4.69%, binder is 3.12% by total weight of intragranulation phase's ingredients, b-) In 40 mg Olmesartan Medoxomil /5 mg amlodipine besylate formulations, intragranulation comprising:

olmesartan medoxomil is 33.02%, amlodipine besylate is 5.72%, poloxamer is 3.47%, filler/diluent is 49.53%, disintegrant is 4.95%, binder is 3.30% by total weight of intragranulation phase's ingredients, c-)In 20 mg Olmesartan Medoxomil /10 mg amlodipine besylate formulations, intragranulation comprising:

olmesartan medoxomil is 16.94%, amlodipine besylate is 11.74%, poloxamer is 1.78%, filler/diluent is 61.07%, disintegrant is 5.08%, binder is 3.39% by total weight of intragranulation phase's ingredients.

**8.** A pharmaceutical composition according to claim 5, wherein said extragranulation step comprises:

disintegrant is in the range of from 1% to 15%, filler/diluent is in the range of from 5% to 80%, glidant is in the range of from 0.1 % to 10%, lubricant is in the range of from 0.1 % to 5%, and coating agent is in the range of from 0.1 % to 10% by total weight of extragranular phase's excipients.

**9.** A pharmaceutical composition according to claim 8, a-) 40 mg olmesartan medoxomil/10 mg amlodipine besylate and 20 mg Olmesartan Medoxomil /5 mg amlodipine besylate formulations, extragranulation comprising: disintegrant is 7.70%, filler/diluent is 76.98%, glidant is 5.70%, lubricant is 1.92 %, coating agent is 7.70% by total weight of extragranulation step's excipients b-)In 40 mg Olmesartan Medoxomil /5 mg amlodipine besylate formulations, extragranulation comprising:

disintegrant is 7.07%, filler is 78.86%, glidant is 5.23%, lubricant is 1.77 %, coating agent is 7.07% by total weight of extragranulation step's excipients,c-)In 20 mg Olmesartan Medoxomil/10 mg amlodipine besylate formulations, extragranulation comprising: disintegrant is 6.82%, filler/diluent is 79.60%, glidant is 5.05%, lubricant is 1.71 %, coating agent is 6.82% by total weight of extragranulation step's excipients.

**10.** A wet granulation process for preparing the pharmaceutical composition according to claim 1, said process comprising the following steps: a. Olmesartan medoxomil and poloxamer are mixed and sieved by using a screen of 315 microns. b. Binder and solvent are mixed until binder is dissolved. c. Mixture prepared at step (a), amlodipine besylate, filler and a portion of disintegrant are mixed in the granulator. d. The granulation solution prepared at step (b) is sprayed onto the mixture in the granulator to form granules. e. The blend is mixed and transferred to a fluidized bed dryer until proper moisture then sizing the dried granules by using suitable milling equipment. f. Granules of internal phase are transferred to the mixer, a portion of disintegrant and additional filler are added to the mixture.g. Pre-sieved glidant and lubricant are added to the mixture and mixed. h. The final blend is compressed on a tabletting machine using appropriate punches. i. Coating agent or mixtures of coating agents are added into purified water and stirred. j. Core tablets are installed into a coating pan and coated with the coating suspension prepared at step (i).

**11.** A direct compression process for preparing the pharmaceutical composition according to claim 1, said process comprising the following steps: a. Olmesartan medoxomil and poloxamer are sieved by using a screen of 315 microns and installed into container and mixed. b. A portion of filler/diluent is added to powder prepared at step (a) and mixed. c. Amlodipine besylate, a portion of filler are sieved by using suitable equipment and mixed. d. A portion of filler/diluent and disintegrant are added to the mixture prepared at step (b) e. The mixture prepared at step (c) and

(d) are mixed f. Pre-sieved glidant and pre-sieved lubricant are installed into container onto powder mixture prepared at step e and mixed. g. The final mixture is compressed h. A Coating agent or mixtures of coating agents are added into solvent and stirred. i. Core tablets are installed into a coating pan and coated with the coating suspension prepared at step h.

**12.** A process according to claim 10 or claim 11 in which olmesartan medoxomil and poloxamer particles are sized with a screen less than 710 microns in terms of intimately admixturing.

## Patentansprüche

**1.** Eine pharmazeutische Zusammensetzung umfassend: Olmesartan Medoxomil in einem Bereich von 5 % bis 45 %, Amlodipine Besylate in einem Bereich von 2 % bis 20 %, Poloxamer in einem Bereich von 0.1 % bis 15 %, Sprengmittel in einem Bereich von 1 % bis 15 %, Füllstoff/Verdünner in einem Bereich von 5 % bis 80 %, Gleitmittel in einem Bereich von 0.1 % bis 10 %, Schmiermittel in einem Bereich von 0.1 % bis 5 %, Beschichtungsmittel in einem Bereich von 0.1 % bis 10 % in Gewichtsanteilen jeweils bezogen auf pharmazeutische Gesamtzusammensetzung, welche durch Verfahren der direkten Kompression oder Feuchtgranulierung hergestellt wird.

**2.** Eine pharmazeutische Zusammensetzung gemäß Anspruch 1 umfassend: Olmesartan medoxomil in einer Menge von 19.42 %, Amlodipine Besylate in einer Menge von 6.73 %, Poloxamer in einer Menge von 2.0 %, Sprengmittel in einer Menge von 4.85 %, Füllstoff/Verdünner in einer Menge von 61.18 %, Gleitmittel in einer Menge von 2.18 %, Schmiermittel in einer Menge von 0.73 %, Beschichtungsmittel in einer Menge von 2.92 % in Gewichtsanteilen, jeweils bezogen auf pharmazeutische Gesamtzusammensetzung.

**3.** Eine pharmazeutische Zusammensetzung gemäß Anspruch 1, hergestellt durch Feuchtgranulierungsverfahren, welche pharmazeutische Zusammensetzung folgendes umfasst: Olmesartan medoxomil in einem Bereich von 5 % bis 45 %, Amlodipine Besylate in einem Bereich von 2 % bis 20 %, Poloxamer in einem Bereich von 0.1 % bis 15 %, Bindemittel in einem Bereich von 0.1 % bis 5 %, Sprengmittel in einem Bereich von 1 % bis 15 %, Füllstoff/Verdünner in einem Bereich von 5 % bis 80 %, Gleitmittel in einem Bereich von 0.1 % bis 10 %, Schmiermittel in einem Bereich von 0.1 % bis 5 %, Beschichtungsmittel in einem Bereich von 0.1 % bis 10 % in Gewichtsanteilen jeweils bezogen auf pharmazeutische Gesamtzusammensetzung.

**4.** Eine pharmazeutische Zusammensetzung gemäß Anspruch 3, a-) in 40 mg Olmesartan Medoxomil/10 mg Amlodipine Besylate und in 20 mg Olmesartan Medoxomil/5 mg Amlodipine Besylate Formulierungen, welche Zusammensetzung umfasst: Olmesartan medoxomil in einer Menge von 19.42 %, Amlodipine Besylate in einer Menge von 6.73 %, Poloxamer in einer Menge von 2.04 %, Bindemittel in einer Menge von 1.94 %, Sprengmittel in einer Menge von 5.82 %, Füllstoff/Verdünner in einer Menge von 58.26 %, Gleitmittel in einer Menge von 2.16 %, Schmiermittel in einer Menge von 0.73 %, Beschichtungsmittel in einer Menge von 2.91 % in Gewichtsanteilen jeweils bezogen auf pharmazeutische Gesamtzusammensetzung, b-) in 40 mg Olmesartan Medoxomil/5 mg Amlodipine Besylate Formulierungen, welche Zusammensetzung enthält: Olmesartan medoxomil in einer Menge von 19.42 %, Amlodipine Besylate in einer Menge von 3.36 %, Poloxamer in einer Menge von 2.04 %, Bindemittel in einer Menge von 1.94 %, Sprengmittel in einer Menge von 5.82 %, Füllstoff/Verdünner in einer Menge von 58.26 %, Gleitmittel in einer Menge von 2.16 %, Schmiermittel in einer Menge von 0.73 %, Beschichtungsmittel in einer Menge von 2.91 % in Gewichtsanteilen, jeweils bezogen auf pharmazeutische Gesamtzusammensetzung, c-) in 20 mg Olmesartan Medoxomil/10 mg Amlodipine Besylate Formulierungen, welche Zusammensetzung enthält: Olmesartan medoxomil in einer Menge von 9.71 %, Amlodipine Besylate in einer Menge von 6.73 %, Poloxamer in einer Menge von 1.02 %, Bindemittel in einer Menge von 1.94 %, Sprengmittel in einer Menge von 5.82 %, Füllstoff/Verdünner in einer Menge von 68.98 %, Gleitmittel in einer Menge von 2.16 %, Schmiermittel in einer Menge von 0.73 %, Beschichtungsmittel in einer Menge von 2.91 % in Gewichtsanteilen jeweils bezogen auf pharmazeutische Gesamtzusammensetzung.

**5.** Eine pharmazeutische Zusammensetzung gemäß Anspruch 3, worin das genannte Feuchtgranulierungsverfahren Intragranulierungs und Extragranulierungsschritte umfasst.

**6.** Eine pharmazeutische Zusammensetzung gemäß Anspruch 5, worin der genannte Intragranulierungsschritt folgendes umfasst: Olmesartan medoxomil in einem Bereich von 5 % bis 45 %, Amlodipine Besylate in einem Bereich von 2 % bis 20 %, Poloxamer in einem Bereich von 0.1 % bis 15 %, Füllstoff/Verdünner in einem Bereich von 5 % bis 80 %, Sprengmittel in einem Bereich von 1 % bis 15 % und Bindemittel in einem Bereich von 0.1 % bis 5 % in

Gewichtsanteilen, jeweils bezogen auf Inhaltsstoffe der intragranularen Phase.

7. Eine pharmazeutische Zusammensetzung gemäß Anspruch 6, a-) in 40 mg Olmesartan Medoxomil/10 mg Amlodipine Besylate und in 20 mg Olmesartan Medoxomil/5 mg Amlodipine Besylate Formulierungen, wobei Intragranulierung folgendes umfasst: Olmesartan medoxomil in einer Menge von 31.24 %, Amlodipine Besylate in einer Menge von 10.82 %, Poloxamer in einer Menge von 3.28 %, Füllstoff/Verdünner in einer Menge von 46.85 %, Sprengmittel in einer Menge von 4.69 %, Bindemittel in einer Menge von 3.12 % in Gewichtsanteilen jeweils bezogen auf Inhaltsstoffe der intragranularen Phase, b-) in 40 mg Olmesartan Medoxomil/5 mg Amlodipine Besylate Formulierungen, wobei Intragranulierung folgendes umfasst: Olmesartan medoxomil in einer Menge von 33.02 %, Amlodipine Besylate in einer Menge von 5.72 %, Poloxamer in einer Menge von 3.47 %, Füllstoff/Verdünner in einer Menge von 49.53 %, Sprengmittel in einer Menge von 4.95 %, Bindemittel in einer Menge von 3.30 % in Gewichtsanteilen jeweils bezogen auf Inhaltsstoffe der intragranularen Phase, c-) in 20 mg Olmesartan Medoxomil/10 mg Amlodipine Besylate Formulierungen, wobei Intragranulierung folgendes umfasst: Olmesartan medoxomil in einer Menge von 16.94 %, Amlodipine Besylate in einer Menge von 11.74 %, Poloxamer in einer Menge von 1.78 %, Füllstoff/Verdünner in einer Menge von 61.07 %, Sprengmittel in einer Menge von 5.08 %, Bindemittel in einer Menge von 3.39 % in Gewichtsanteilen jeweils bezogen auf Inhaltsstoffe der intragranularen Phase.

8. Eine pharmazeutische Zusammensetzung gemäß Anspruch 5, worin der genannte Extragranulierungsschritt folgendes umfasst: Sprengmittel in einem Bereich von 1 % bis 15 %, Füllstoff/Verdünner in einem Bereich von 5 % bis 80 %, Gleitmittel in einem Bereich von 0.1 % bis 10 %, Schmiermittel in einem Bereich von 0.1 % bis 5 % und Beschichtungsmittel in einem Bereich von 0.1 % bis 10 % in Gewichtsanteilen jeweils bezogen auf das Gesamtgewicht der Hilfsstoffe der extragranularen Phase.

9. Eine pharmazeutische Zusammensetzung gemäß Anspruch 8, a-) in 40 mg Olmesartan Medoxomil/10 mg Amlodipine Besylate und in 20 mg Olmesartan Medoxomil/5 mg Amlodipine Besylate Formulierungen, wobei Extragranulierung folgendes umfasst: Sprengmittel in einer Menge von 7.70 %, Füllstoff/Verdünner in einer Menge von 76.98 %, Gleitmittel in einer Menge von 5.70 %, Schmiermittel in einer Menge von 1.92 %, Beschichtungsmittel in einer Menge von 7.70 in Gewichtsanteilen jeweils bezogen auf das Gesamtgewicht der Hilfsstoffe des extragranularen Schritts, b-) in 40 mg Olmesartan Medoxomil/5 mg Amlodipine Besylate Formulierungen, wobei Extragranulierung folgendes umfasst: Sprengmittel in einer Menge von 7.07 %, Füllstoff in einer Menge von 78.86 %, Gleitmittel in einer Menge von 5.23 %, Schmiermittel in einer Menge von 1.77 %, Beschichtungsmittel in einer Menge von 7.07 in Gewichtsanteilen jeweils bezogen auf das Gesamtgewicht der Hilfsstoffe des extragranularen Schritts, c-) in 20 mg Olmesartan Medoxomil/10 mg Amlodipine Besylate Formulierungen, wobei Extragranulierung folgendes umfasst: Sprengmittel in einer Menge von 6.82 %, Füllstoff/Verdünner in einer Menge von 79.60 %, Gleitmittel in einer Menge von 5.05 %, Schmiermittel in einer Menge von 1.71 %, Beschichtungsmittel in einer Menge von 6.82 in % Gewichtsanteilen jeweils bezogen auf das Gesamtgewicht der Hilfsstoffe des extragranularen Schritts.

10. Ein Feuchtgranulierungsverfahren zur Herstellung der pharmazeutischen Zusammensetzung gemäß Anspruch 1, wobei das genannte Verfahren die folgenden Schritten umfasst: a. Olmesartan Medoxomil und poloxamer werden vermischt und durch ein 315-Mikron-Sieb gesiebt. b. Bindemittel und Lösemittel werden bis zur kompletten Auflösung des Bindemittels vermischt. c. Die Mischung aus Schritt (a), Amlodipine Besylate, Füllstoff und ein Teil von Sprengmittel werden in einem Granulator vermischt. d. Die Granulationslösung aus Schritt (b) wird auf die Mischung im Granulator besprüht, um ein Granulat daraus zu erhalten. e. Das Gemisch wird gerührt und zu einem Wirbelschichttrockner überführt bis zum Erreichen eines geeigneten Feuchtegehaltes, anschließend wird das getrocknete Granulat mit Hilfe einer geeigneten Mahlvorrichtung auf die gewünschte Größe gebracht. f. Das Granulat aus der inneren Phase wird zu einem Rührwerk überführt, es werden der Mischung ein Teil von Sprengmittel und zusätzlicher Füllstoff zugegeben und gemischt. g. Vorgesiebtes Gleitmittel und Schmiermittel werden der Mischung zugegeben und gerührt. h. Das Endgemisch wird auf einer Tablettiermaschine unter Einsatz von geeigneten Stanzen komprimiert. i. Beschichtungsmittel oder Mischungen davon werden in demineralisiertes Wasser gegeben und gerührt. j. Kerntabletten werden auf eine Beschichtungspfanne gelegt und mit einer im Schritt (i) vorbereiteten Beschichtungssuspension beschichtet.

11. Ein Direktkomprimierungsverfahren zur Herstellung der pharmazeutischen Zusammensetzung gemäß Anspruch 1, wobei das genannte Verfahren die folgenden Schritte umfasst: a. Olmesartan Medoxomil und poloxamer werden durch ein 315-Mikron-Sieb gesiebt, in einen Behälter gebracht und gemischt. b. Ein Teil von Füllstoff/Verdünner wird dem Pulver aus (a) zugegeben und vermischt. c. Amlodipine Besylate und ein Teil von Füllstoff werden unter Einsatz von einer geeigneten Vorrichtung gesiebt. und vermischt. d. Der Mischung aus (b) werden ein Teil von Füllstoff/Verdünner und Sprengmittel zugegeben. e. Die Mischungen aus Schritt (c) und (d) werden untereinander

vermischt. f. Vorgesiebtes Gleitmittel und vorgesiebtes Schmiermittel werden in den Behälter gebracht und auf die Pulvermischung aus Schritt (e) gegeben und vermischt. g. Das Endgemisch wird komprimiert h. Ein Beschichtungsmittel oder Mischungen davon werden ins Lösemittel gegeben und gerührt. i. Kerntabletten werden auf eine Beschichtungspfanne gelegt und mit einer im Schritt (h) vorbereiteten Beschichtungssuspension beschichtet.

**12.** Ein Verfahren gemäß Anspruch 10 oder Anspruch 11, worin die Partikeln von Olmesartan Medoxomil und Poloxamer, im Sinne einer innigen Vermischung, unter Einsatz von einem Sieb auf eine Größe unterhalb 710 Mikron gebracht werden.

**Revendications**

**1.** Composition pharmaceutique comprenant: l'olmesartan medoxomil se situe dans l'intervalle de 5% à 45%, le bésylate d'amlodipine se situe dans l'intervalle de 2% à 20%, le poloxamère se situe dans l'intervalle de 0,1% à 15%, le désintégrant se situe dans l'intervalle de 1% à 15%, le remplisseur/diluant se situe dans l'intervalle de 5% à 80%, l'agent de glissement se situe dans l'intervalle de 0,1% à 10%, le lubrifiant se situe dans l'intervalle de 0,1% à 5%, l'agent d'enrobage se situe dans l'intervalle de 0,1% à 10% en poids de la composition pharmaceutique totale préparée par les méthodes de compression directe ou de granulation humide.

**2.** Composition pharmaceutique selon la revendication 1, comprenant: l'olmesartan medoxomil est 19,42%, le bésylate d'amlodipine est 6,73%, le poloxamère est 2,00%, le désintégrant est 4,85%, le remplisseur/diluant est 61,18 %, l'agent de glissement est 2,18%, le lubrifiant est 0,73%, l'agent d'enrobage est 2,92% en poids de la composition pharmaceutique totale.

**3.** Selon la revendication 1, composition pharmaceutique préparée par la méthode de granulation humide dans laquelle ladite composition pharmaceutique comprenant: l'olmesartan medoxomil se situe dans l'intervalle de 5% à 45%, le bésylate d'amlodipine se situe dans l'intervalle de 2% à 20%, le poloxamère se situe dans l'intervalle de 0,1% à 15%, le liant se situe dans l'intervalle de 0,1% à 5%, le désintégrant se situe dans l'intervalle de 1% à 15%, le remplisseur/diluant se situe dans l'intervalle de 5% à 80%, l'agent de glissement se situe dans l'intervalle de 0,1% à 10%, le lubrifiant se situe dans l'intervalle de 0,1% à 5%, l'agent d'enrobage se situe dans l'intervalle de 0,1% à 10% en poids de la composition pharmaceutique totale.

**4.** Composition pharmaceutique selon la revendication 3, a) dans les formulations de l'Olmesartan Medoxomil, 40 mg / le bésylate d'amlodipine, 10 mg et de l'Olmesartan Medoxomil, 20 mg / le bésylate d'amlodipine, 5 mg, la composition pharmaceutique comprenant: l'olmesartan medoxomil est 19,42%, le bésylate d'amlodipine est 6,73%, le poloxamère est 2,04%, le lient est 1,94%, le désintégrant est 5,82%, le remplisseur/diluant est 58,26 %, l'agent de glissement est 2,16%, le lubrifiant est 0,73%, l'agent d'enrobage est 2,91% en poids de la composition pharmaceutique totale; b) dans les formulations de l'Olmesartan Medoxomil, 40 mg / le bésylate d'amlodipine, 5 mg, la composition pharmaceutique comprenant: l'olmesartan medoxomil est 19,42%, le bésylate d'amlodipine est 3,36%, %, le poloxamère est 2,04%, le lient est 1,94%, le désintégrant est 5,82%, le remplisseur/diluant est 58,26%, l'agent de glissement est 2,16%, le lubrifiant est 0,73%, l'agent d'enrobage est 2,91% en poids de la composition pharmaceutique totale; c) dans les formulations de l'Olmesartan Medoxomil, 20 mg / le bésylate d'amlodipine, 10 mg, la composition pharmaceutique comprenant: l'olmesartan medoxomil est 9,71%, le bésylate d'amlodipine est 6,73%, le poloxamère est 1,02%, le lient est 1,94%, le désintégrant est 5,82%, le remplisseur/diluant est 68,98%, l'agent de glissement est 2,16%, le lubrifiant est 0,73%, l'agent d'enrobage est 2,91% en poids de la composition pharmaceutique totale.

**5.** Composition pharmaceutique selon la revendication 3, dans laquelle ladite méthode de granulation humide comprend les étapes d'intragranulation et d'extragranulation.

**6.** Composition pharmaceutique selon la revendication 5 , dans laquelle ladite étape d'intragranulation comprend: l'olmesartan medoxomil se situe dans l'intervalle de 5% à 45%, le bésylate d'amlodipine se situe dans l'intervalle de 2% à 20%, le poloxamère se situe dans l'intervalle de 0,1% à 15%, le remplisseur/diluant se situe dans l'intervalle de 5% à 80%, le désintégrant se situe dans l'intervalle de 1% à 15%, le liant se situe dans l'intervalle de 0,1% à 5% en poids des ingrédients de l'étape intragranulaire.

**7.** Composition pharmaceutique selon la revendication 6, a) dans les formulations de l'Olmesartan Medoxomil, 40 mg / le bésylate d'amlodipine, 10 mg et de l'Olmesartan Medoxomil, 20 mg / le bésylate d'amlodipine, 5 mg, l'intragranulation comprenant: l'olmesartan medoxomil est 31,24%, le bésylate d'amlodipine est 10,82%, le poloxamère est

3,28%, le remplisseur/diluant est 46,85%, le désintégrant est 4,69%, le lient est 3,12% en poids total des ingrédients de l'étape d'intragranulation; b) dans les formulations de l'Olmesartan Medoxomil, 40 mg / le bésylate d'amlodipine, 5 mg, l'intragranulation comprenant: l'olmesartan medoxomil est 33,02%, le bésylate d'amlodipine est 5,72%, %, le poloxamère est 3,47%, le remplisseur/diluant est 49,53%, le désintégrant est 4,95%, le lient est 3,30%, en poids total des ingrédients de l'étape d'intragranulation; c) dans les formulations de l'Olmesartan Medoxomil, 20 mg /le bésylate d'amlodipine, 10 mg, l'intragranulation comprenant: l'olmesartan medoxomil est 16,94%, le bésylate d'amlodipine est 11,74%, le poloxamère est 1,78%, le remplisseur/diluant est 61,07%, le désintégrant est 5,08%, le lient est 3,39% en poids total des ingrédients de l'étape d'intragranulation.

**8.** Composition pharmaceutique selon la revendication 5, dans laquelle ladite étape d'extragranulation comprend: le désintégrant se situe dans l'intervalle de 1% à 15%, le remplisseur/diluant se situe dans l'intervalle de 5% à 80%, l'agent de glissement se situe dans l'intervalle de 0,1% à 10%, le lubrifiant se situe dans l'intervalle de 0,1% à 5% et l'agent d'enrobage se situe dans l'intervalle de 0,1% à 10% en poids total des ingrédients de l'étape extragranulaire.

**9.** Composition pharmaceutique selon la revendication 8, a) dans les formulations de l'Olmesartan Medoxomil, 40 mg / le bésylate d'amlodipine, 10 mg et de l'Olmesartan Medoxomil, 20 mg / le bésylate d'amlodipine, 5 mg, l'extragranulation comprenant: le désintégrant est 7,70%, le remplisseur/diluant est 76,98%, l'agent de glissement est 5,70%, le lubrifiant est 1,92%, l'agent d'enrobage est 7,70% en poids total des ingrédients de l'étape d'extragranulation; b) dans les formulations de l'Olmesartan Medoxomil, 40 mg /le bésylate d'amlodipine, 5 mg, l'extragranulation comprenant: le désintégrant est 7,07%, le remplisseur est 78,86%, l'agent de glissement est 5,23%, le lubrifiant est 1,77%, l'agent d'enrobage est 7,07% en poids total des ingrédients de l'étape d'extragranulation; c) dans les formulations de l'Olmesartan Medoxomil, 20 mg / le bésylate d'amlodipine, 10 mg, l'extragranulation comprenant: le désintégrant est 6,82%, le remplisseur/diluant est 79,60%, l'agent de glissement est 5,05%, le lubrifiant est 1,71%, l'agent d'enrobage est 6,82% en poids total des ingrédients de l'étape d'extragranulation.

**10.** Procédé de granulation humide pour préparer la composition pharmaceutique selon la revendication 1, ledit procédé comprenant les étapes suivantes: a. L'olmesartan medoxomil et le poloxamère sont mélangés et tamisés en utilisant un tamis de 315 microns d'ouverture de maille. b. Le liant et le solvant sont mélangés jusqu'à ce que le liant soit dissous. c. La mixture préparée à l'étape (a), le bésylate d'amlodipine, le remplisseur et une portion du désintégrant sont mélangés dans le granulateur. d. La solution de granulation préparée à l'étape (b) est pulvérisée sur la mixture dans le granulateur pour former des granules. e. Le mélange est mêlé et transféré au sécheur à lit fluidisé jusqu'à l'humidité appropriée, puis, les tailles des granules séchés sont mesurées en utilisant un équipement de broyage approprié. f. Les granules de la phase interne sont transférés au mélangeur, une portion du désintégrant et le remplisseur additionnel sont ajoutés à la mixture. g. L'agent de glissement pré-tamisé et le lubrifiant sont ajoutés à la mixture et mélangés. h. Le mélange final est compressé sur une machine de production de comprimés en utilisant des poinçons appropriés. i. L'agent d'enrobage ou les mixtures d'agents d'enrobage sont ajoutés à l'eau purifiée et remués. j. Les comprimés-noyaux sont installés dans une cuve d'enrobage et enrobés avec la suspension d'enrobage préparée à l'étape (i).

**11.** Procédé de compression directe pour préparer la composition pharmaceutique selon la revendication 1, ledit procédé comprenant les étapes suivantes: a. L'olmesartan medoxomil et le poloxamère sont tamisés en utilisant un tamis de 315 microns d'ouverture de maille et installés dans le container et mélangés. b. Une portion du remplisseur/diluant est ajoutée à la poudre préparée à l'étape (a) et mélangée. c. Le bésylate d'amlodipine, une portion du remplisseur sont tamisés en utilisant un équipement approprié et mélangés. d. Une portion du remplisseur/diluant et désintégrant sont ajoutés à la mixture préparée à l'étape (b). e. La mixture préparée à l'étape (c) et (d) sont mélangées. f. L'agent de glissement pré-tamisé et le lubrifiant pré-tamisé sont installés dans le container sur la mixture de poudre préparée à l'étape (e) et mélangés. g. Le mélange final est compressé. h. L'agent d'enrobage ou les mixtures d'agents d'enrobage sont ajoutés au solvant et remués. i. Les comprimés-noyaux sont installés dans une cuve d'enrobage et enrobés avec la suspension d'enrobage préparée à l'étape (h).

**12.** Procédé selon la revendication 10 ou la revendication 11, dans lequel les tailles de particules de l'olmesartan medoxomil et du poloxamère sont mesurées en utilisant un tamis de moins de 710 microns d'ouverture de maille en termes de mélange intime.

[Fig. 1]

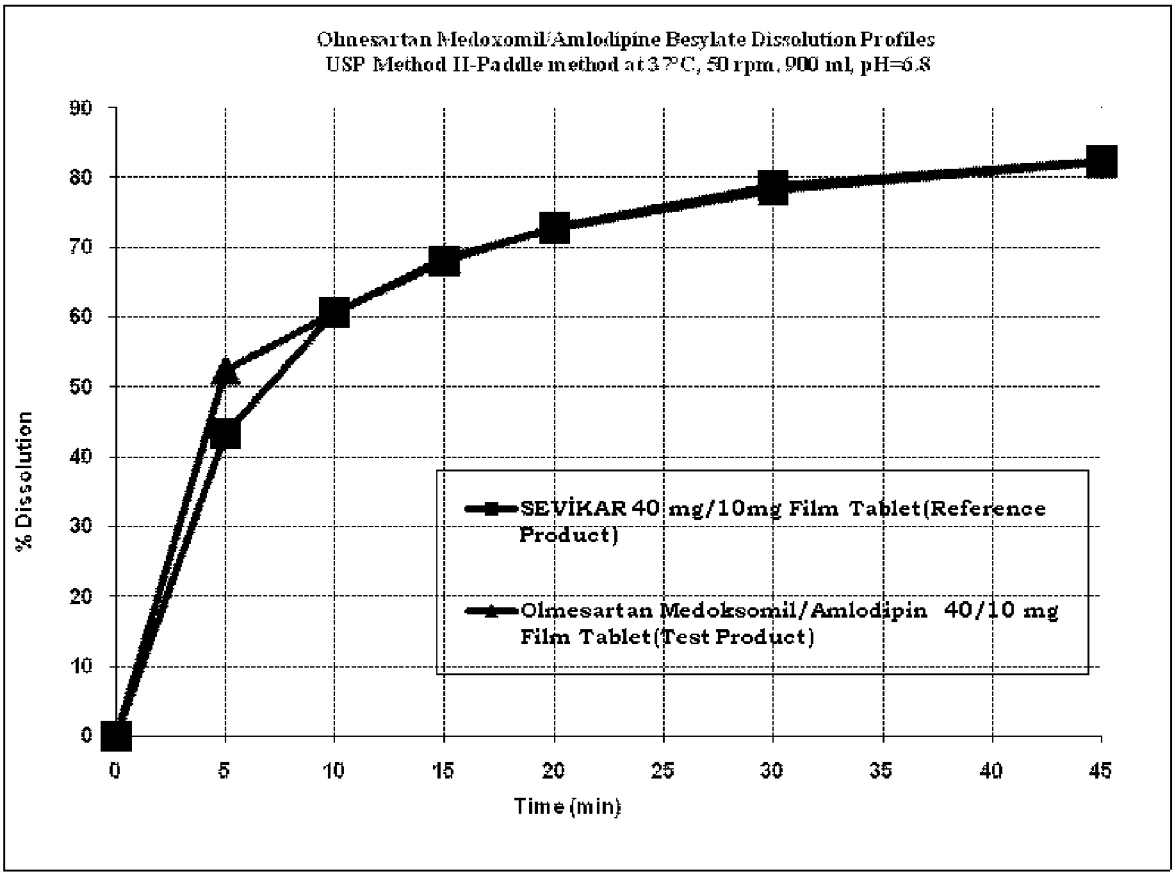
Olmesartan Medoxomil/Amlodipine Besylate Dissolution Profiles
USP Method II-Paddle method at 37°C, 50 rpm, 900 ml, pH=6.8
% Dissolution
Time (min)
0
10
20
30
40
50
60
70
80
90
0
5
10
15
20
25
30
35
40
45
SEVİKAR 40 mg/10mg Film Tablet(Reference Product)
Olmesartan Medoksomil/Amlodipin 40/10 mg Film Tablet(Test Product)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0503785 B
- EP 244944 B
- WO 2008149338 A
- EP 2033643 A
- WO 2007001066 A
- WO 2007001065 A
- WO 2008032107 A
- US 20100119607 A
- WO 2009084040 A
- WO 20080131 A